# EUROPEAN PATENT SPECIFICATION

(11) **EP 0 597 101 B1**
(45) Date of publication and mention of the grant of the patent: **24.02.1999**
(21) Application number: 92915866.5
(22) Date of filing: 17.07.1992
(51) Int. Cl.: A61K 39/395, A61K 9/14, A61K 47/18, A61K 47/26

(54) **STABILIZED HUMAN MONOCLONAL CLN-IgG ANTIBODY PREPARATION**
STABILISIERTE AUFBEREITUNG MONOKLONALER MENSCHLICHER CLN-IgG ANTIKÖRPER
PREPARATION A ANTICORPS MONOCLONAL HUMAIN CLN-IgG STABILISE

(30) Priority: 20.07.1991 JP 204743/91
(43) Date of publication of application: 18.05.1994
(73) Proprietor: Hagiwara, Yoshihide, Takarazuka-shi Hyogo-ken (JP)
(72) Inventor: HAGIWARA, Hideaki, Takarazuka-shi, Hyogo 665 (JP); YUASA, Hideo, Kasai-shi, Hyogo 675-22 (JP); YAMAMOTO, Yasunori, Kasai-shi, Hyogo 675-23 (JP)
(74) Representative: Weisert, Annekäte, Dipl.-Ing. Dr.-Ing.
(86) International application number: JP9200914
(87) International publication number: WO9301835

(56) References cited:
- EP-A- 0 410 207
- JP-A-53 047 515
- JP-A-56 127 320
- JP-A-63 088 197

## Description

This invention relates to a stabilized human monoclonal antibody preparation, and more detailedly, relates to a human monoclonal antibody preparation excellent in stability a solution state, a freeze drying state and a freezing state, particularly redissolution (restoration) stability after freezing drying.

### Background Art

Since a process for producing a monoclonal antibody by genetic engineering was proposed in 1975 by Koehler and Milstein [Koehler, G., Milstein, C, Nature 256, 495 (1975)], a road has been opened up to supply a large quantity of a monoclonal antibody as a homogeneous antibody, and such monoclonal antibodies have widely been utilized in the medical and biological fields.

Recently, human monoclonal antibodies are provided in human clinical tests, and particularly draw attention in the antitumor-directed medicinal field. However, purified human monoclonal antibodies have an undesirable property as a preparation that they easily aggregate and precipitate in a solution state or at the time of redissolution (restoration) after freeze drying, and development of monoclonal antibody preparations having no such an undesirable property and being stabilized is desired.

On the other hand, as methods for stabilization of antibodies (immunoglobulins), there have hitherto been proposed a method which comprises adding to a sulfonated immunoglobulin serum albumin, or serum albumin with glycine and/or mannitol (Japanese Patent Publication No. 20965/1987); a method which comprises adding a comparatively large quantity of a polyhydric alcohol (Japanese Laid-Open Patent Publication No. 88197/1988); a method for the stabilization of a polyclonal antibody by using D-mannitol resulting in a relatively low solubility of the antibody after freeze-drying (JP-A-56/127320); a method for the stabilization of highly purified proteins such as murine monoclonal antibodies, which comprises the addition of carbohydrates such as mannitol during the purification process (EP-A-0 410 207); a method for the stabilization of polyclonal antibodies by using glucides such as glucose or fructose (JP-A-53/47515); a method which comprises adding dextran (Japanese Laid-Open Patent Publication No. 225320/1988). However, it is impossible, by these so far proposed methods, to improve sufficiently the above undesirable property in human monoclonal antibody preparations, especially in compositions comprising CLN-IgG, which is producible by Hybridoma CLN H11 (ATCC HB 8307) (in the following referred to as "CLN-IgG"). The present inventors now found that stability of a human monoclonal antibody composition comprising CLN-IgG, particularly stability against aggregation and precipitation at the time of redissolution after freeze drying of the human monoclonal antibody CLN-IgG is remarkably enhanced by compounding a specified small quantity of mannitol to the human monoclonal antibody CLN-IgG, and completed this invention.

Thus, there is provided according to this invention a stabilized human monoclonal antibody composition comprising human monoclonal antibody CLN-IgG and from 1 to 20 mg of D-mannitol per 1 mg of CLN-IgG.

The human monoclonal antibody CLN-IgG can be made into preparations for putting to practical use as medicinal drugs, etc. As a process for making into a preparation, there can, for example, be mentioned a process which comprises, according to necessity, concentrating the purified human monoclonal antibody CLN-IgG by ultrafiltration, sodium sulfate fractionation or the like, substituting a buffer solution suitable for the preparation by a gel filtration method, in some case further adjusting the concentration, making a filtration sterilization processing, and then making freeze drying.

In preparation of a stabilized human monoclonal antibody composition comprising CLN-IgG, it is possible to compound D-mannitol as a stabilizer at any stage of the above making of a preparation, but generally, it is suitable to introduce D-mannitol into a human monoclonal antibody composition comprising CLN-IgG by a dialytic method after substitution with a buffer solution suitable for a preparation by a gel filtration method. The concentration of D-mannitol in a D-mannitol solution usable for the dialytic method differs depending on the concentration of a human monoclonal antibody solution comprising CLN-IgG to be dialyzed, and for example is suitable in the range of generally 0.1 to 2% (w/v), preferably 0.5 to 1.5% (w/v) in case the concentration of the human monoclonal antibody CLN-IgG is 1 mg/ml, and suitable in the range of generally 0.1 to 10% (w/v), preferably 0.5 to 5% (w/v) in case the concentration of the human monoclonal antibody CLN-IgG is 5 mg/ml.

The content of D-mannitol can be in the range of 1 to 20 mg, preferably 5 to 15 mg per 1 mg of the human monoclonal antibody CLN-IgG in the preparation. When the content of D-mannitol is smaller than 1 mg, a desired sufficient stabilization effect cannot be obtained, and when it is larger than 20 mg, agglutination of the antibody comes conversely to be observed.

Further, it was revealed that stability of the preparation was further enhanced by using glycine in addition to D-mannitol. Although the use quantity of glycine at that time is not strictly restricted, but it is suitable that the use quantity is in the range of generally 0.005 to 0.2 mole, preferably 0.1 to 0.15 mole per 1 mg of the human monoclonal antibody CLN-IgG.

Introduction of glycine into the preparation of this invention can be made at the same time of introduction of D-mannitol.

Further if necessary, it is possible to compound a suitable quantity of a phosphate salt or the like for adjustment of pH into the preparation of this invention.

### Example

This invention is further specifically described below according to examples.

### Reference example 1 : Preparation of human monoclonal antibody CLN-IgG

Freezed cells of an antibody producing cell [human x human hybridoma = CLN H11 (ATCC HB 8307)] were thawed, and the thawed cells were washed with a basal medium and then cultured using a basal medium containing 10% fetal bovine serum. After culture, the cells were taken from this culture broth and cultured again in a serum-free medium (Hybrity-II, produced by HIH Biocenter Co. located at Kasai-shi, Hyogo-ken, Japan), and then scale up was made by batch culture in the same medium. Cells were removed from 40 liters of the resultant serum-free culture broth, and the resultant solution was concentrated to about 5 liters by ultrafiltration (PROSTAK™, produced by Millipore Co.).

Salting-out was carried out by adding ammonium sulfate to the concentrate so that the final concentration of the saturated solution became 70% to obtain a precipitate with ammonium sulfate.

This ammonium sulfate precipitate was dialyzed twice against 20 liters each of 10 mM phosphate buffer solution (hereafter referred to as PB) for total 24 hours, and then adsorbed on a cation exchange column (S-Sepharose, fast flow, produced by Pharmacia Co.). The column adsorbate was sufficiently washed with 10 mM PB, and then eluted by an NaCl concentration gradient from 0 to 0.5 M in 10 mM PB to obtain a rough fraction of IgG.

This was adsorbed on a Protein A column (produced by Repligen Co.), and after sufficient washing with 10 mM PB + 1 M NaCl, eluted with 0.1 M glycine-hydrochloric acid + 1M NaCl (pH 3.0).

The resultant IgG was concentrated by ammonium sulfate fractionation (saturation concentration 50%), and the concentrate was subjected to gel filtration using a Sephacryl S-300 column (produced by Pharmacia Co.) equilibrated with 10 mM phosphate-buffered physiological saline (hereafter referred to as PBS) to obtain purified IgG.

### Reference example 2 : Preparation of stabilizer, etc.

(1) The phosphate-buffered physiological saline (PBS) was prepared by dissolving 1.15 g of Na₂HPO₄ (anhydrous), 8.0 g of NaCl, 0.2 g of KH₂PO₄ and 0.2 g of KCl in about 900 ml of distilled water, adjusting the pH to 7.2 to 7.4, and making the total quantity 1.0 liter.
(2) As the physiological saline for injection was used one produced by Otsuka Pharmaceutical Co.
(3) The mannitol solutions were prepared by diluting 20% (w/v) D-mannitol injection produced by Otsuka Pharmaceutical Co. with distilled water to concentrations of 1%, 5% and 10% (w/v), respectively.
(4) 1% mannitol + physiological saline for injection was prepared by dissolving D-mannitol in physiological saline for injection to make the concentration 1% (w/v).
(5) The glycine-mannitol solution was prepared by dissolving 22.5 g of glycine, 50 ml of 20% D-mannitol solution and 1.56 g of NaH₂PO₄· 2H₂O in about 900 ml of water and adjusting the pH to 7.2 to 7.4, and making the total quantity 1.0 liter.

### Example 1

### Preparation and stability of freeze dried agents of the human monoclonal antibody CLN-IgG ― (1)

The human monoclonal antibody solution prepared in Reference example 1 was dialyzed against each solution prepared in Reference example 2. The resultant each human monoclonal antibody solution was adjusted to each concentration of 1.0, 2.5 and 5.0 mg/ml. The resultant solutions were passed through a membrane filter of 0.22 µm, and 1 ml portions thereof were poured into vials and freeze dried using a tray dryer produced by LABCONCO Co., USA. Freeze drying was made by holding the samples at a shelf temperature of -30°C for about three hour for freezing, and after complete freezing of the samples, starting drying by moving a suction pump. The shelf temperature was raised to 0°C, and about 20 hours later, the freeze drying was finished.

1 ml portions of distilled water were added to the vials, respectively to dissolve freeze dried powders, and solubilities were compared based on OD₆₀₀ values usually used in measurement of the turbidity of culture broths of bacteria, etc. When insoluble particles were formed as a result of aggregation of the antibody, etc., OD₆₀₀ values increase. As a result, it was revealed, as shown in the following Table 1, that the 1% (w/v) mannitol solution and the glycine-mannitol solution are the best in view of solubility after freeze drying of the human monoclonal antibody. Further, even in case of the solutions of mannitol alone, the solubility went bad in the solutions having high concentrations of 5% and 10% (w/v). Further, even when the concentration of mannitol was 1% (w/v), existence in 0.9% or so of NaCl made the solubility worse, as shown in the result of 1% mannitol + physiological saline for injection.

**Table 1**

| Solubility (OD₆₀₀) of human monoclonal antibody CLN-IgG after freeze drying in each stabilizer | | | | |
|---|---|---|---|---|
| Stabilizer | Quantity (mg) of antibody in one vial | | | |
| | 0 | 1.0 | 2.5 | 5.0 |
| PBS | 0.019 | 0.194 | 0.311 | 0.427 |
| Physiological saline for injection | 0.001 | 0.220 | 0.582 | 0.952 |
| 1% mannitol | 0.001 | 0.014 | 0.035 | - |
| 5% mannitol | 0.000 | 0.194 | 0.270 | - |
| 10% mannitol | 0.001 | 0.246 | 0.317 | - |
| 1% mannitol + physiological saline for injection | 0.001 | 0.193 | 0.228 | - |
| Glycine-mannitol | 0.010 | 0.042 | - | 0.115 |

### Example 2

### Preparation and stability of freeze dried agents of the human monoclonal antibody CLN-IgG ― (2)

According to the method described in Example 1, freeze dried agents were prepared containing in one vial 1, 2, 5, 10, 15, 20, 50 or 100 mg of D-mannitol and 1, 2.5 or 5 mg of the monoclonal antibody, and solubilities were compared. The results are shown in Table 2.

As a result, it was revealed that when the quantity of D-mannitol after freeze drying is in the range of 1 to 20 mg per 1 mg of the antibody, sufficient solubility can be obtained.

**Table 2**

| Solubility (OD₆₀₀) of human monoclonal antibody CLN-IgG after freeze drying in D-mannitol | | | |
|---|---|---|---|
| Quantity (mg) of D-mannitol one vial | Quantity (mg) of antibody in one vial | | |
| | 1 mg | 2.5 mg | 5 mg |
| 1 | 0.007 | 0.008 | 0.008 |
| 2 | 0.005 | 0.004 | 0.013 |
| 5 | 0.003 | 0.003 | 0.002 |
| 10 | 0.001 | 0.004 | 0.008 |
| 15 | 0.002 | 0.002 | 0.005 |
| 20 | 0.005 | 0.012 | 0.006 |
| 50 | 0.194 | 0.022 | 0.024 |
| 100 | 0.246 | 0.127 | 0.031 |

### Industrial Applicability

As stated above, the human monoclonal antibody composition comprising CLN-IgG of this invention is excellent in stability in a solution state, a freeze drying state and a freezing state, particularly stability against aggregation and precipitation of the human monoclonal antibody at the time of redissolution after freeze drying, and is useful as a medicinal drug.

## Claims

1. A stabilized human monoclonal antibody composition comprising human monoclonal antibody CLN-IgG producible by Hybridoma CLN H11 (ATCC HB 8307) and from 1 to 20 mg of D-mannitol per 1 mg of CLN-IgG.

2. The preparation according to claim 1 containing 5 to 15 mg of D-mannitol per 1 mg of the human monoclonal antibody.

3. The preparation according to claim 1 further containing glycine.

4. The preparation according to claim 3 containing 0.005 to 0.2 mole of glycine per 1 mg of the human monoclonal antibody.

5. The preparation according to claim 4 containing 0.1 to 0.15 mole of glycine per 1 mg of the human monoclonal antibody.

6. A process for stabilizing a human monoclonal antibody composition comprising CLN-IgG, producible by Hybridoma CLN H11 (ATCC HB 8307) which comprises dialyzing the human monoclonal antibody in a buffer solution against a D-mannitol solution, wherein a final concentration of D- mannitol of from about 1 to 20 mg per mg of antibody is obtained in the buffer solution.

7. The process according to claim 6 wherein the D-mannitol solution comprises 0.1 to 2 % (w/v) D-mannitol, and wherein the concentration of the human monoclonal antibody is 1 mg/ml.

8. The process according to claim 6 wherein the D-mannitol solution comprises 0.1 to 10 % (w/v) D-mannitol, and wherein the concentration of the human monoclonal antibody is 5 mg/ml.

## Patentansprüche

1. Stabilisierte humane monoclonale Antikörperzusammensetzung, die den humanen monoclonalen Antikörper CLN-IgG, der von dem Hybridom CLN H11 (ATCC HB 8307) hergestellt werden kann, und 1 bis 20 mg D-Mannit je 1 mg CLN-IgG umfaßt.

2. Zubereitung nach Anspruch 1, dadurch **gekennzeichnet,** daß sie 5 bis 15 mg D-Mannit je 1 mg des humanen monoclonalen Antikörpers enthält.

3. Zubereitung nach Anspruch 1, dadurch **gekennzeichnet,** daß sie zusätzlich Glycin enthält.

4. Zubereitung nach Anspruch 3, dadurch **gekennzeichnet,** daß sie 0,005 bis 0,2 mol Glycin je 1 mg des humanen monoclonalen Antikörpers enthält.

5. Zubereitung nach Anspruch 4, dadurch **gekennzeichnet,** daß sie 0,1 bis 0,15 mol Glycin je 1 mg des humanen monoclonalen Antikörpers enthält.

6. Verfahren zur Stabilisierung einer humanen monoclonalen Antikörperzusammensetzung, die CLN-IgG, der von dem Hybridom CLN H11 (ATCC HB 8307) hergestellt werden kann, enthält, umfassend das Dialysieren des humanen monoclonalen Antikörpers in einer Pufferlösung gegen eine D-Mannitlösung, wobei eine Endkonzentration von D-Mannit von etwa 1 bis 20 mg je mg Antikörper in der Pufferlösung erhalten wird.

7. Verfahren nach Anspruch 6, dadurch **gekennzeichnet,** daß die D-Mannitlösung 0,1 bis 2% (Gew./Vol.) D-Mannit enthält und daß die Konzentration des humanen monoclonalen Antikörpers 1 mg/ml beträgt.

8. Verfahren nach Anspruch 6, dadurch **gekennzeichnet,** daß die D-Mannitlösung 0,1 bis 10% (Gew./Vol.) D-Mannit enthält und daß die Konzentration des humanen monoclonalen Antikörpers 5 mg/ml beträgt.

## Revendications

1. Composition stabilisée d'un anticorps monoclonal humain comprenant un anticorps monoclonal humain CLN-IgG que l'on peut produire par l'hybridome CLN H11 (ATCC HB 8307) et de 1 à 20 mg de D-mannitol pour 1 mg de CLN-IgG.

2. Préparation selon la revendication 1, contenant de 5 à 15 mg de D-mannitol pour 1 mg de l'anticorps monoclonal humain.

3. Préparation selon la revendication 1, contenant de plus de la glycine.

4. Préparation selon la revendication 3, contenant de 0,005 à 0,2 mole de glycine pour 1 mg de l'anticorps monoclonal humain.

5. Préparation selon la revendication 4, contenant de 0,1 à 0,15 mole de glycine pour 1 mg de l'anticorps monoclonal humain.

6. Procédé pour la stabilisation d'une composition d'anticorps monoclonal humain comprenant CLN-IgG, que l'on peut produire par l'hybridome CLN H11 (ATCC HB 8307), lequel comprend la dialyse de l'anticorps monoclonal humain dans une solution tampon contre une solution de D-mannitol, dans laquelle on obtient une concentration finale en D-mannitol d'environ 1 à 20 mg par mg d'anticorps dans la solution tampon.

7. Procédé selon la revendication 6, où la solution de D-mannitol comprend de 0,1 à 2 % (w/v) de D-mannitol, et où la concentration en anticorps monoclonal humain est de 1 mg/ml.

8. Procédé selon la revendication 6, où la solution de D-mannitol comprend de 0,1 à 10 % (w/v) de D-mannitol et où la concentration en anticorps monoclonal humain est de 5 mg/ml.
